# EUROPEAN PATENT APPLICATION

(11) **EP 4 830 677 A1**
(43) Date of publication of application: **28.10.2026**
(21) Application number: 26165819.9
(22) Date of filing: 18.03.2026
(51) Int. Cl.: A61F 7/00

(54) **APPLICATOR FOR BEAUTY TREATMENTS**

(30) Priority: 23.04.2025 IT 202500009084
(71) Applicant: Biotec S.r.l., 36031 Dueville (Vicenza) (IT)
(72) Inventor: FERRARI, Fulvio, 35123 PADOVA (PADOVA) (IT)
(74) Representative: Forattini, Amelia

(57) **Abstract**

An applicator for beauty treatments, comprising a substantially hollow body defining at least one chamber provided with an opening facing outwards and configured to be placed in contact with a skin tissue to be treated; the chamber is delimited by at least one lateral surface and comprises at least one suction mouth susceptible to be connected to suction means, which are capable of creating a depression that enables to suction the skin tissue into the same chamber, so as to make it adhere to at least a portion of the lateral surface; the applicator comprises at least one insufflation opening configured to deliver a flow of gaseous fluid between the lateral surface and the skin tissue, without hindering the operation of the suction means.

## Description

The present invention relates to an applicator for beauty treatments, particularly for cryolipolysis treatments.

The invention also relates to an apparatus comprising said applicator.

As is well known, beauty treatments include a wide range of procedures aimed at improving physical appearance without necessarily resorting to surgery. They are generally divided into facial treatments and body treatments and may have anti-ageing, reshaping, purifying or firming purposes.

More specifically, facial treatments are mainly aimed at improving the quality of the skin, reducing wrinkles, blemishes and other signs of ageing.

Body treatments, on the other hand, generally act on localised fat deposits, cellulite, skin firmness, and hydration.

In the field of body treatments, one of the most popular non-invasive techniques for reducing localised fat deposits without surgery is cryolipolysis. This technique is based on the principle that fat cells are more sensitive to low temperatures than other tissues.

In practice, during a cryolipolysis treatment, the area of the body to be treated is cooled in a controlled manner, inducing the crystallisation of fat cells; this leads to the rupture of the membranes of the cells, causing their death. The dead fat cells are then naturally eliminated from the body over the following weeks.

In general, cryolipolysis treatments are carried out using special applicators that, by a suction system, create a vacuum effect that sucks in the skin and the underlying fat tissue in order to form a skin fold. This skin fold is then cooled until the fat cells are frozen without damaging the skin, muscles or nerves.

Cryolipolysis treatments do not require surgery or anaesthesia and are generally considered to be safe; however, they may involve some side effects and complications.

The most common side effects are short-term and may include redness, swelling and bruising caused by the vacuum and cooling. In addition, numbness and reduced sensitivity may occur, which may last from a few days to a few weeks, and even mild pain, which is manageable with mild analgesics.

In some cases, however, cryolipolysis treatments can also be responsible for the onset of a serious complication known as PAH (Paradoxical Adipose Hyperplasia).

Paradoxical adipose hyperplasia generally consists of an abnormal increase and hardening of fat cells in the treated area following treatment.

The exact causes are unclear, but it is thought that some fat cells, instead of dying, may enter a hyperactive state, increasing in volume, due to a temperature that is too low or to an excessive exposure time during treatment.

This condition, which generally manifests itself in the formation of a hard, protruding, well-defined mass under the skin, which follows the shape of the applicator used, is unfortunately irreversible without surgery.

US2016/038183A1 discloses a method of treating a skin surface of a subject comprising heating a skin surface, abrading native skin tissue of a subject using a microdermabrasion device, wherein using the microdermabrasion device comprises moving the microdermabrasion device relative to the skin surface while simultaneously delivering at least one treatment fluid to the skin surface being treated and cooling the abraded skin surface.

US2024/082051A1 discloses an apparatus for cooling an object, space, or tissues of a patient. A vacuum chamber is designed to be placed in thermal contact with the object or space to be cooled, or against a patient to be treated. A water sprayer is configured to spray water into the vacuum chamber or against a cooling wall of the chamber. A vacuum pump and control are designed to maintain vacuum below ambient pressure in the vacuum chamber sufficient to cause accelerated evaporation of the water and cooling to a temperature desired for cooling of the object, space, or patient.

WO2014/097288A2 discloses an apparatus comprising an array of individually controlled skin treatment units to be applied to a skin segment with each of the units comprising: a housing defining a cavity that fluidly communicates with a source of vacuum pressure, having an inner side terminated by a rim facilitating sealing of the cavity when the unit is applied to the skin, dimensions of the cavity being sufficient to accommodate a volume of a skin segment drawn into the cavity by the source of vacuum pressure to create a skin protrusion; wherein application and release of vacuum pressure to the unit generates a back and forth massaging movement of at least a portion of a volume of skin against the rim, and wherein each of the units of the array has at least two degrees of rotational movement with respect to an adjacent unit so that the array can conform to skin segment topography.

US2016/256671A1 discloses a device and method for treating the skin using a rollerball or a wicking member. The skin treatment assembly comprises a handpiece comprising a distal end, the handpiece comprising a recess or cavity configured to receive a cartridge or other fluid container, a tip configured to be positioned along the distal end of the handpiece, the tip being configured to contact a skin surface of a subject during use, and at least one rollerball configured to extend to or near the tip, wherein the rollerball is configured to be in fluid communication with an interior of a cartridge or other fluid container secured to the handpiece, wherein the at least one rollerball is configured to contact the skin surface of the subject during use and to facilitate the delivery of fluids to said skin surface as the rollerball is moved relative to said skin surface.

The aim of the present invention is to provide an applicator for beauty treatments that overcomes the drawbacks of the known technique mentioned above.

Within the scope of this object a particular aim of the invention is to provide an applicator that, in cryolipolysis treatments, enables the effective prevention of paradoxical adipose hyperplasia.

A further object of the invention is to provide an applicator that, compared to prior art devices for cryolipolysis treatments, allows to reduce the discomfort caused to the patient by cooling.

A further object of the invention is to provide a versatile applicator that can also be advantageously used to implement beauty treatments other than cryolipolysis, with various purposes, such as treatments generating a massaging effect or involving the application of different forms of energy, not necessarily combined with cooling.

A further object of the invention is to provide an applicator that is safe and effective.

This aim and these and other objects which will become better apparent hereinafter are achieved by a base assembly for electrical boxes as claimed in the appended claims.

Further characteristics and advantages will become better apparent from the description of preferred but not exclusive embodiments of the invention, illustrated by way of non-limiting example in the accompanying drawings, wherein:

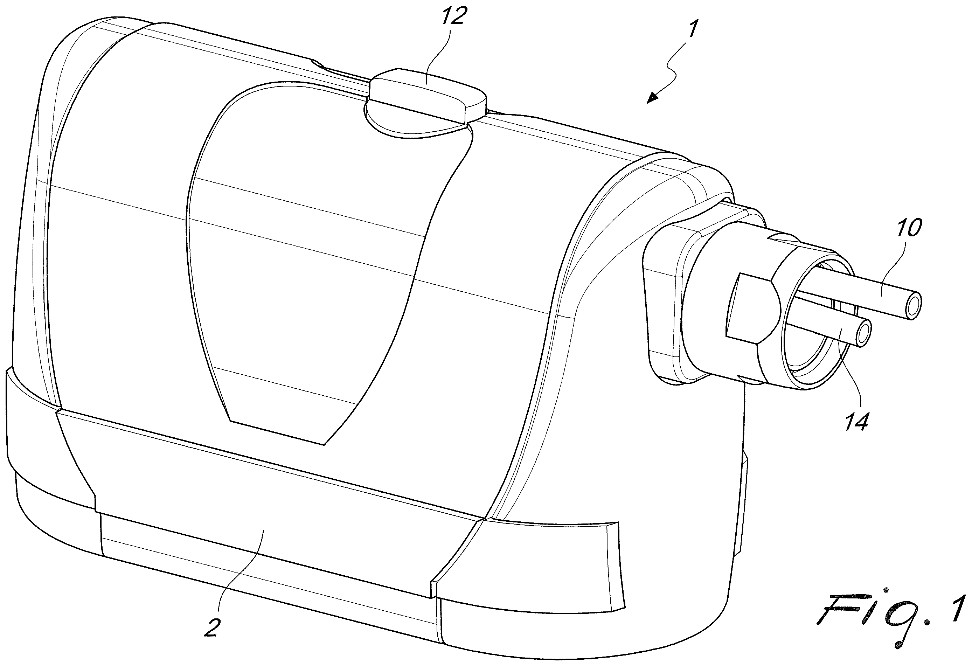

Figure 1 is a perspective view of an applicator for beauty treatment according to the invention;

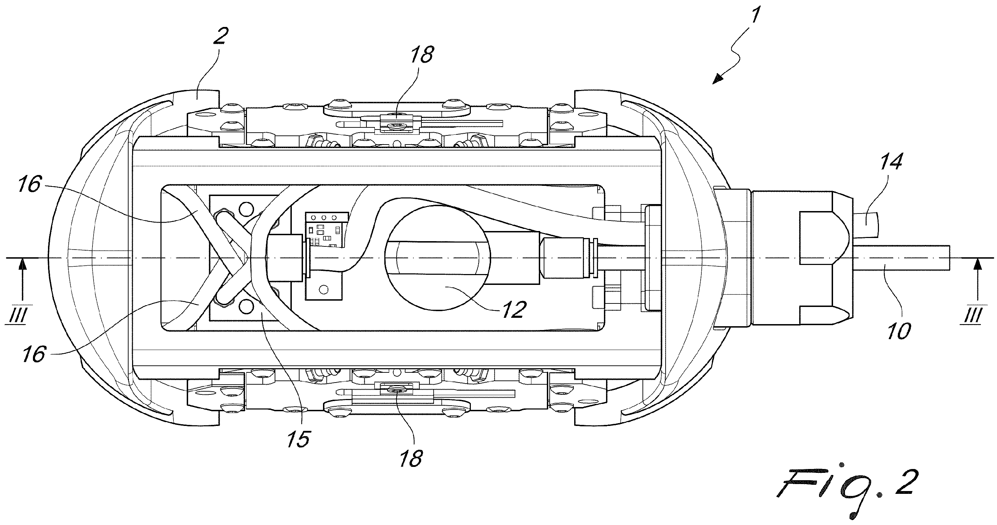

Figure 2 is a top view of the applicator according to the invention;

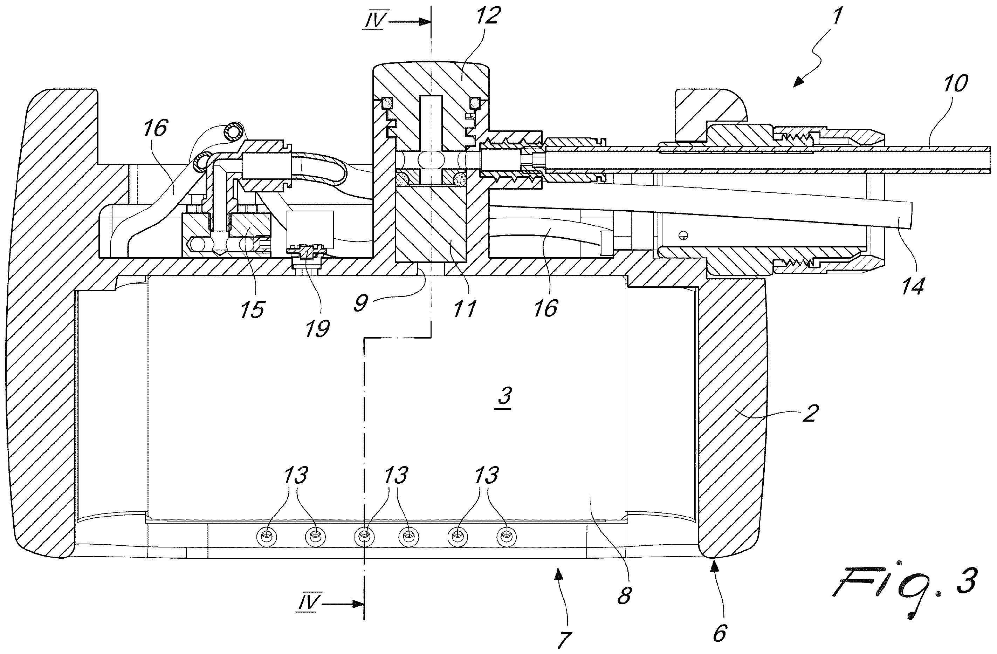

Figure 3 is a section along line III-III of figure 2;

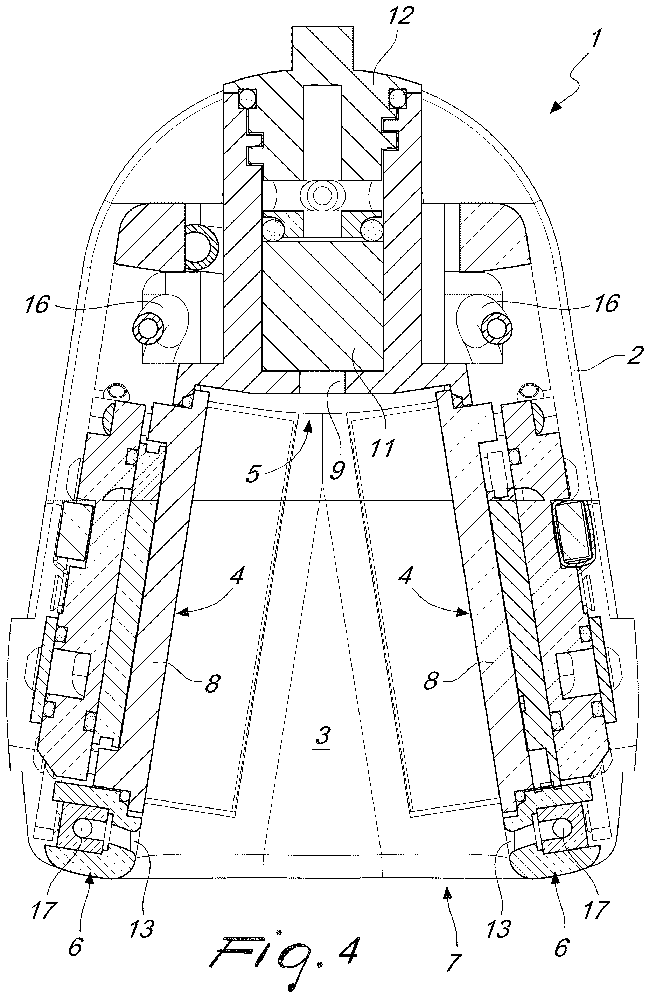

Figure 4 is a section along line IV-IV of figure 3;
Figure 5 is a sectional view of the applicator shown during a beauty treatment.

With reference to the figures, an applicator for beauty treatments, hereafter also referred to as an "applicator", is globally designated by the reference number 1.

The applicator 1 comprises a substantially hollow body 2 forming a chamber 3 receiving and treating skin tissue 100 affected by blemishes, such as localised fat deposits.

Chamber 3 has a lateral surface 4, continuous or discontinuous, which extends between a sky surface 5 and an edge 6 surrounding an opening 7.

The opening 7 faces outwards and is configured to be positioned in mechanical contact with the skin tissue 100.

Advantageously, the lateral surface 4 is at least partially formed by a pair of plates 8, opposed to each other, made of thermally and electrically conductive material.

Preferably, the plates 8 diverge towards the opening 7 and, together with the sky surface 5, form a volume in the chamber 3, said volume having a substantially pyramidal or truncated-pyramidal geometry.

A suction mouth 9 is opened in the chamber 3; the suction mouth 9 is adapted to be connected to suction means, not illustrated, that create a depression configured to suck part of the skin tissue 100 in the chamber 3, forming a fold with the skin and with the underlying panniculus adiposus and to make this fold at least partially adhere to the plates 8.

Preferably, the suction means comprise a vacuum pump, not illustrated, and vacuum regulating means, also not illustrated, connected to the pump and allowing a regulation of the depression it exerts within the chamber 3.

Advantageously, the suction means are connected to the chamber 3 by at least a first tube 10, of a flexible type. A filter 11 is arranged at one end of the first tube 10 and prevents contamination of the suction means with any biological fluids from the skin tissue 100.

Advantageously, the filter 11 is selectively accessible from the outside by means of a cap 12, for example of the screw type.

The applicator 1 may comprise a sealing member, not illustrated in the figures, associated with the edge 6 delimiting the opening 7, in order to maintain an isolation between the chamber 3 and the outside, when the edges 6 rest on the skin tissue 100.

The sealing member may be made of a plastic material, preferably non-toxic, unalterable, suitable for sterilization and autoclaving.

The applicator 1 comprises a plurality of insufflation openings 13 configured to deliver, on command and selectively, a flow of gaseous fluid, e.g. air, into the chamber 3, directing such flow between the lateral surface 4 and the skin tissue 100.

Advantageously, the gaseous fluid may be delivered in a pulsed manner, i.e. according to a uniform and paced sequence, or in a rhythmic manner, i.e. according to a more articulated and dynamic sequence.

According to a preferred embodiment of the invention, the insufflation openings 13 comprise a plurality of through-holes distributed substantially uniformly over an inner portion of the edge 6.

Said through-holes are configured to be connected to gaseous fluid insufflation means, not illustrated, via a second tube 14, of a flexible type.

More particularly, the second tube 14 is connected to a manifold 15 which, by means of tubes 16, conveys the gaseous fluid towards channels 17 obtained in the body 2 and placed in fluid communication with the insufflation openings 13.

The applicator 1 further comprises temperature variation means 18 associated with the chamber 3 and configured to receive energy from a power unit, not illustrated, and, on command and selectively, to cool the skin tissue 100 through the lateral surface 4.

According to a preferred embodiment of the invention, the temperature variation means 18 comprise one or more Peltier cells associated with the plates 8 and adapted to be connected to the power unit.

According to further embodiments of the invention, the temperature variation means comprise a refrigeration circuit, wherein a heat transfer fluid circulates.

The applicator 1 may also comprise power supplying means associated with the chamber 3 and configured to receive power from the power unit and deliver, on command and selectively, electrical stimulation to the skin tissue 100 via the lateral surface 4.

According to a preferred embodiment of the invention, the plates 8 deliver the electrical energy and also operate as electrodes in order to deliver electrical current pulses to the skin tissue 100.

The applicator 1 further comprises at least one distance sensor 19, preferably of an optoelectronic type, associated with the chamber 3 and configured to detect the distance between the skin tissue 100 and the sky surface 5 of the chamber 3.

By means of the distance sensor 19, which interacts with a control unit, not illustrated, it is possible to check whether the fold comprising the skin and the underlying panniculus adiposus is correctly accommodated in the chamber 3 and to control the vacuum adjustment means accordingly.

The operation of the applicator according to the invention is as follows.

First, the applicator 1 is connected to an apparatus comprising the suction means, the insufflation means, the supply unit and the control unit described above.

Once the applicator 1 is connected to the apparatus, the optimal positioning of the body 2 with respect to the user's body is sought, so as to ensure the correct support of the opening 7 on the skin tissue 100, a necessary condition in order to apply the vacuum inside the chamber 3.

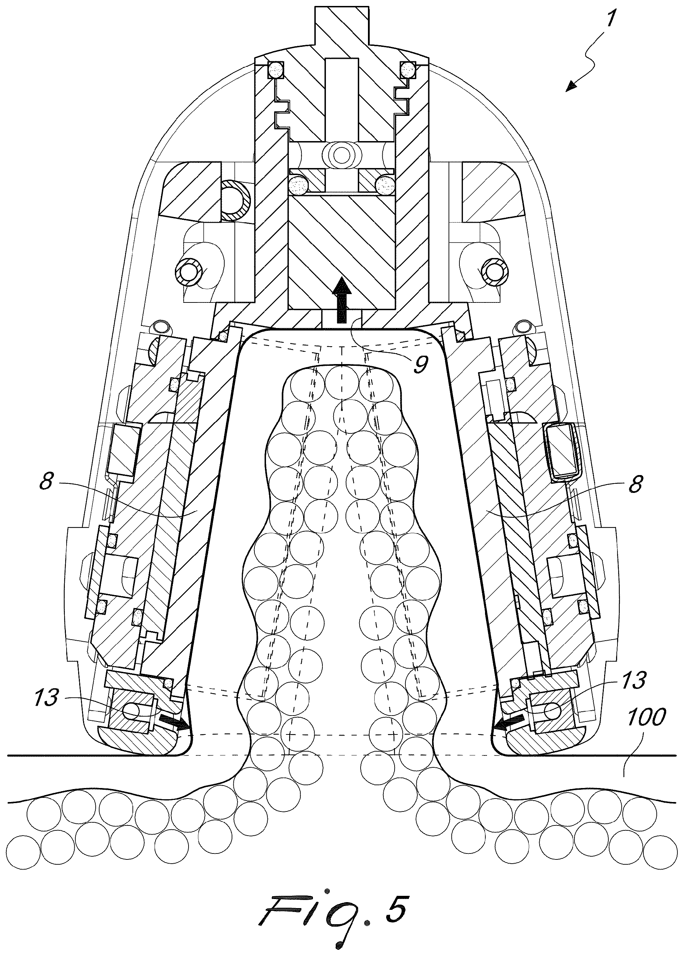

At this point, the chamber 3 is isolated from the outside, so that, by activating the vacuum pump, a depression is created inside the same chamber 3, which ensures the adhesion of the fold formed by the skin and the underlying panniculus adiposus to the plates 8, as illustrated in figure 5.

By means of the adjustment means, it is possible to modify the degree of depression generated inside the chamber 3, adapting it to the specific treated area and thus making the fold formed by the skin tissue 100 adhere correctly to the plates 8; this adjustment is facilitated by the presence of the distance sensor 19 and its interaction with the control unit.

After having correctly positioned the applicator 1 on the skin tissue 100, the temperature variation means 18 and/or the means supplying electrical energy are actuated.

Simultaneously, the insufflation means are operated thus delivering, through the insufflation openings 13, a rhythmic or pulsed flow of gaseous fluid between the plates 8 and the skin tissue 100, interrupting the continuity of contact between them.

In cryolipolysis treatments, the cyclic touching and separating of the skin tissue 100 and the plates 8 reduces the exposure time of the fat cells to a temperature which, under certain conditions, may be too low; this prevents trauma to the user and, in particular, makes it possible to prevent or significantly reduce the likelihood of paradoxical adipose hyperplasia occurring.

It should be noted, however, that the insufflation means do not necessarily have to act in combination with the temperature variation means 18 or even with the power delivery means; the insufflation means may also be used independently, in order to obtain a beneficial massaging effect on the skin tissue 100.

Once the treatment is completed, the depression exerted inside the chamber 3 is interrupted, after which the applicator 1 can be easily removed from the skin tissue 100 of the user.

It has been found in practice that the invention achieves its intended aims and objects by an applicator for beauty treatments which, by delivering a rhythmic or pulsed flow of fluid between the skin tissue to be treated and the applicator, makes it possible to prevent, or to considerably reduce the occurrence of paradoxical adipose hyperplasia in cryolipolysis treatments.

According to an aspect of the invention, the distance sensor 19 interacting with the control unit makes it possible to check whether the fold comprising the skin and the underlying panniculus adiposus is correctly accommodated in the chamber 3, thus controlling the vacuum adjustment means.

Without proper contact with the skin, the formation of micro-air bubbles inside the suction cavity can cause localized overheating, potentially resulting in burns or injury.

The sensor according to the present invention effectively solves the problem of preventing such occurrences, ensuring treatment safety.

Compared to prior art devices, the present invention also significantly reduces the discomfort caused to the patient by the cooling applied during cryolipolysis treatments.

According to a further advantage of the present invention, the applicator can also be advantageously used for beauty treatments involving the application of different forms of energy, such as electric pulses, not necessarily combined with cooling. In this case, the massaging effect that the pulsed or rhythmic fluid flow induces on the skin tissue is mainly used for a draining and/or toning effect.

## Claims

1. An applicator (1) for beauty treatments, comprising a substantially hollow body (2) forming at least one chamber (3) having an opening (7) facing outwards and configured to be placed in contact with a skin tissue (100) to be treated; said chamber (3) being delimited by at least one lateral surface (4) and comprising at least one suction mouth (9) configured to be connected to suction means that form a depression that makes said skin tissue (100) to be sucked into said chamber (3), making said skin tissue (100) adhere to at least a portion of said lateral surface (4); said applicator further comprising at least one insufflation opening (13) configured to deliver a flow of gaseous fluid between said lateral surface (4) and said skin tissue (100), without hindering the operation of said suction means; said applicator being **characterized in that** it comprises at least one distance sensor (19) associated with said chamber (3), said distance sensor (19) being configured to detect the distance between said skin tissue (100) and a sky surface (5) of said chamber (3).

2. The applicator according to the preceding claim, **characterized in that** it comprises a plurality of insufflation openings (13) configured to be connected to insufflation means of said gaseous fluid; said insufflation openings (13) being distributed substantially uniformly over an inner portion of an edge (6) delimiting said opening (7).

3. The applicator according to one or more of the preceding claims, **characterized in that** said insufflation openings (13) are in fluid communication with one or more channels (17) formed in said body (2); said channels (17) being connected to a manifold (15) by means of pipes (16); said manifold (15) being selectively supplied by said insufflation means with said gaseous fluid.

4. The applicator according to one or more of the preceding claims, **characterized in that** said gaseous fluid is delivered into said chamber (3) in a pulsed or rhythmic manner.

5. The applicator according to claim 1, **characterized in that** it comprises temperature variation means (18) associated with said chamber (3), said temperature variation means (18) being configured to receive energy from a supply unit and cool said skin tissue (100) through said lateral surface (4).

6. The applicator according to the preceding claim, **characterized in that** said temperature variation means (18) comprise one or more Peltier cells associated with said lateral surface (4).

7. The applicator according to claim 1, **characterized in that** it comprises means supplying electrical energy associated with said chamber (3); said means supplying electrical energy being configured to receive energy from said power supply unit and provide electrical stimulation to said skin tissue (100) via said lateral surface (4).

8. The applicator according to one or more of the preceding claims, **characterized in that** said lateral surface (4) is at least partially formed by a pair of plates (8), opposed to each other, associated with said chamber (3); said plates (8) being made of thermally and/or electrically conductive material.

9. The applicator according to one or more of the preceding claims, **characterized in that**, during operation, said skin tissue (100) in said chamber (3) forms a fold at least partially adhering to said plates (8); said plates (8) operating as electrodes delivering pulses of electric current to said skin tissue (100) by said means supplying electric energy.

10. The applicator according to one or more of the preceding claims, **characterized in that** said lateral surface (4) is at least partially divergent towards said opening (7).

11. The applicator according to one or more of the preceding claims, **characterized in that** it comprises a sealing member associated with each of said edges (6) delimiting said opening (7); said sealing member improving isolation between said chamber (3) and the outside, when said edges (6) rest on said skin tissue (100).

12. The applicator according to the preceding claim, **characterized in that** said distance sensor (19) is of an optoelectronic type.

13. An apparatus for beauty treatments, according to one or more of the preceding claims, **characterized in that** it comprises suction means, insufflation means, at least one power supply unit, at least one control unit and at least one said applicator (1); said at least one applicator (1) being operatively connectable to said suction means, to said insufflation means, to said at least one power supply unit, and to said at least one control unit.
